# EUROPEAN PATENT APPLICATION

(11) **EP 1 930 412 A1**
(43) Date of publication of application: **11.06.2008**
(21) Application number: 06025095.8
(22) Date of filing: 05.12.2006
(51) Int. Cl.: C12N 5/06, A61K 35/12

(54) **Neural stem cell medium and differentiation method**

(71) Applicant: Dialectica s.r.l., 20014 Nerviano MI (IT)
(72) Inventor: Cattaneo, Elena, 20047 Brugherio (MI) (IT); Conti, Luciano, 23851 Galbiate (LC) (IT); Reitano, Erika, 20135 Milano (IT); Goffredo, Donato, 20011 Corbetta (MI) (IT)
(74) Representative: Banfi, Paolo

(57) **Abstract**

Neural stem cells are differentiated to form neurons using a medium containing a combination of a neurotrophin, an FGF receptor agonist and a glial differentiation inhibitor. Neural stem cells are preconditioned prior to transplantation so that the preconditioned cells have a marker profile which is nestin negative, Tuj1 positive, doublecortin positive, N-CAM positive, ki67 negative and phosphohistone 3 negative.

## Description

The present invention relates to neural stem cells, culture conditions and methods of culturing neural stem cells (NS cells or NSCs) in order to promote neuronal differentiation and to precondition cells to promote integration of neurons after transplantation into the brain. Compositions, cell preparations and single neural stem cells are also provided.

Neural stem cells are commonly isolated from multiple sources with the aim of deriving *in vitro* cell cultures showing stable neurogenesis over time. However, these cultures are characterised by low levels of neuronal differentiation and poor survival rates in long-term culture. Consequently these cells are not suitable for reliable drug testing and researchers have been reliant on primary cell cultures.

One of the most widely used methods of culturing neural stem cells utilises neurospheres. This system produces heterologous aggregates of cells principally consisting of committed progentitors and differentiated cells with a very low proportion of stem cells.

The heterogeneity of the neurosphere population prevents any meaningful characterisation of the stem cell portion (Suslov et al 2002). Additionally, gene expression profiling of clonal neurosphere cell lines has found large differences between the different cell lines and was unable to provide a definition of stem cells within neurospheres (Suslov at el 2002).

The neurosphere system has proven unable to maintain large scale cultures of neural stem cells in a symmetrically dividing, undifferentiated state.

Alternatively, adherent cell cultures have been derived, however, the high tendency of the neural stem cells to differentiate has limited large scale cultures to a lifespan of 15-20 passages.

Attempts to stabilise these cultures have resulted in the development of various media and supplement formulations. For example, culturing neural stem cells in media containing a combination of Epidermal Growth Factor (EGF) and Fibroblast Growth Factor-2 (FGF-2) is widely known in the art. Work on primary neural progenitors derived from embryonic stem cells established that FGF-2 induces neural progenitor proliferation (Okabe et al 1996, Li et al 1998) but that neurogenic capacity was progressively lost in both neurosphere (Itsykson et al 2005) and adherent (Brustle et al 1999) cultures. Addition of EGF supported the expansion of a distinct subset of neural progenitors which acquired a homogeneous morphology and marker expression that characterised them as neural stem cells (Ying et al 2003).

Neural stem cells have also been derived from mouse foetal forebrain when primary cell clusters or long term passaged neurospheres have been allowed to settle in EGF plus FGF-2 (Conti et al 2005). In all essential features these cells are indistinguishable from those that have been derived from embryonic stem cells.

Successful transplantation of neural stem cells into the brain has the potential to offer effective treatment for a variety of neurological disorders, requiring a stable population of highly plastic NS cells that are capable of responding to *in vivo* environmental cues. A previous study by Conti et al showed that preconditioned transplanted neural stem cells will survive in the adult mouse brain but these cells differentiated into a mixture of neurons and glial cells, did not show any region specific differentiation and did not develop into fully mature cells (Conti et al 2005).

NS cell cultures in the prior art show 50% type III beta-tubulin at day 10 of culture with 20% survival after two weeks in culture. It is desired to increase the % expression of type III beta-tubulin and/or the % survival in culture.

The prior art cells are able to differentiate but due to their poor survival there are few neurons in culture - these tend to detach and die, making the cells difficult to characterise. The cultures additionally show high levels of contamination.

The *in vitro* neurons are not like *in vivo* neurons, in that not all endogenously expressed receptors are present and they not functionally active. This makes them unsuitable for drug testing which is consequently limited to primary cultures.

Neural stem cells transplanted into the brain differentiate into a mixture of immature neurons and glial cells with no region specific variation, making them unsuitable for therapeutic use. It is known to attempt to precondition cells for transplantation by 7 day cultures in the presence of laminin. These preconditioned cells, when transplanted, do not integrate well and do not show regio-specific differentiation.

It is an object of the invention to improve the differentiation of neural stem cells into neurons, leading typically to increased numbers of functional neurons. A further object is to improve integration of transplanted neural stem cells and progeny thereof. An object of specific embodiments of the invention is increased survival of neurons and provision of *in vivo,* transplanted neural cells with regio-specific morphology.

Accordingly the present invention provides a method of culture of neural stem cells so as to produce neurons, a method of preconditioning of neural stem cells prior to transplantation, uses of the cells and media and kits therefor.

A first aspect of the invention provides a method of differentiating neural stem cells so as to form neurons, comprising culturing said cells in medium containing a combination of a neurotrophin, an FGF receptor agonist and a glial differentiation inhibitor.

In use, the combination promotes differentiation into neurons, and it is found that omission of any one of these components will compromise the survival, the quality and the efficiency of neuronal differentiation. Omission of glial inhibitor induced a higher percentage of glial cells (more than 10-15% vs 5% or less when all components are present). Additionally there is a reduced quality of the neurons generated, both in terms of morphology (less elongated neurons and with a less complex neuronal morphology i.e. mainly bipolar neurons) and antigenic maturation (lower expression of mature neuronal markers, like map2 and Na+ channels) without the glial differentiation inhibitor. Omission of FGF receptor agonist resulted in accelerated cell death with cultures principally comprising a small number of glial cells after 10 days. Thus, the full combination showed improved cell survival. Omission of BDNF induced cells to begin detaching easily after 10 days and undergo cell death. Additionally the neurons that did develop did not show proper morphological and antigenic maturation.

The neurotrophin is suitably selected from BDNF, NGF, NT3 and NT4, and is preferably BDNF. Several FGF receptor agonists are also suitable such as FGF1, FGF2, FGF3, FGF4 and FGF8. In preferred embodiments FGF-2 is used.

A glial differentiation inhibitor is advantageously used to reduce the proportion of neural stem cells that form glial cells in culture. In examples below, higher than expected proportions of cells forming functional neurons, with reduced glial contamination have been obtained. Typical glial differentiation inhibitors are selected from Trichostatin A, sodium butyrate, suberoylanilide hydroxamic acid, suberoyl-3-aminopyridineamide hyroxamic acid and MS-275, and a particularly preferred inhibitor is valproic acid.

In carrying out the cultures of the invention, optionally divided into distinct culture stages or steps, cell density is preferably controlled within certain limits. For the differentiation methods, it is preferred to plate neural stem cells at a density of 30,000 cells/cm² or less, more preferably at a density of from 5,000 cells/cm² to 25,000 cells/cm² and further preferably at a density of from 10,000 cells/cm² to 22,000 cells/cm². In specific examples, the density is from 14,000 cells/cm² to 18,000 cells/cm².

Differentiation methods of the invention are generally comprised of two or more stages, and in embodiments of the invention there is a first culture step and a second culture step, wherein the second culture step is a method as described above and the first culture step comprises culturing the cells in the absence of a neurotrophin, in the absence of a glial differentiation inhibitor and in the presence of an agonist of an FGF receptor. This two or more stage process is found to lead to high levels of neuronal differentiation in the absence of production of significant contamination of the cultures.

Plating neural stem cells in the first culture step can be done at a density of at least 50,000 cells/cm² , preferably from 50,000 cells/cm² to 200,000 celIS/_{CM}², more preferably from 80,000 cells/cm² to 150,000 cells/cm².

The second culture step can further include or be sub-divided into a first period of culture and a second period of culture, and wherein during the first period the cells are cultured in a ratio of neurotrophin:FGF receptor agonist of from 1:1 to 6:1 1 and during the second period the cells are cultured in a ratio of neurotrophin:FGF receptor agonist of from 7:1 to 20:1. This change in relative concentration of these factors is believed also to contribute to the increased proportion of neurons obtained.

Culture time is also typically maintained with certain limits. In the first period of the second culture step the cells can be cultured for from 1 to 5 days, preferably from 2 to 4 days, more preferably for about 3 days, meaning in this and other contexts +/- 6 hours.

Similarly, during the first culture step, the cells can be cultured for from 1 to 5 days, preferably from 2 to 4 days, more preferably for about 3 days.

In a second aspect, the invention provides a method of differentiating neural stem cells, the method comprising:-
(a) culturing neural stem cells in medium containing a neurotrophin and an FGF receptor agonist at a first ratio;
(b) culturing the cells in a medium containing a neurotrophin and an FGF receptor agonist at a second ratio,
   wherein the second ratio is at least twice the first ratio.

Hence during culture so as to promote neuron formation the relative amount of the neurotrophin increases at least two fold.

In a preferred method, neural stem cells are cultured in medium containing a neurotrophin and an FGF receptor agonist at a ratio of 1-6:1; and then at a ratio of 7-20:1.

In adjusting the relative amounts of neurotrophin and FGF receptor agonist between (a) and (b), it is preferred to reduce the absolute amount of FGF receptor agonist, to promote neuron function. We have observed that if the concentration of the FGF receptor agonist is not decreased, the culture does not become post-mitotic and a significant number of cells are still proliferating after 7 days in culture, thus resulting in a higher culture density on day 10. This results in reduced neuronal differentiation both in terms of efficiency and neuronal quality. The reduction in concentration also allows the cells to reach a more enhanced and late neuronal maturation stage. The increasing concentration of neurotrophin compensates for the negative effect of reducing the FGF receptor agonist, as the neurotrophin acts as a potent neuronal survival factor.

We further observed that the FGF receptor agonist cannot be completely eliminated as the initial health of the cultures is unfavourably affected and the neuronal inductive effect of the medium used in a first step is compromised. If the FGF receptor agonist is completely removed from later step(s) the attachment of cells after replating and survival in the days following differentiation is negatively affected.

Optional and preferred features of this aspect of the invention are as described above with reference to the first aspect of the invention.

In a third aspect of the invention, there is provided a method of differentiating neural stem cells, the method comprising:
(a) culturing neural stem cells at a density of at least 50,000 cells/cm ²
(b) re-plating said cells at a density of 5000 - 30,000 cells/CM².

There is thus a significant reduction in the plating density over these two culturing steps. In use of the invention, if the plating density of the cells is too low survival of the cells will be greatly affected. If the plating density is too high the efficiency of neuronal differentiation is greatly reduced.

During step (a) the cells are typically cultured for a period of from 1 to 5 days. During step (b) the cells are typically cultured for a period of from 1 to 5 days. Optional and preferred features of this aspect of the invention are as described above with reference to the first aspect of the invention.

In particularly preferred embodiments of the invention, neural stem cells are converted into neurons by:
(a) culturing neural stem cells at a density of 100,000 - 120,000 cells/cm² in NS-A medium containing N2 supplement, B27 supplement and FGF-2 for from 2 to 4 days;
(b) dissociating the cells and re-plating the cells at a density of 14,000 - 18,000 cells/cm² in neurobasal medium (DMEM-F 12), containing N2 supplement, B27 supplement, FGF-2, BDNF and valproic acid and culturing said cells for from 2 to 4 days; and
(c) transferring said cells to neurobasal medium (DMEM-F 12), containing N2 supplement, B27 supplement, FGF-2, BDNF and valproic acid.

In a specific embodiment of the invention, the method comprises:
(a) culturing neural stem cells at a density of 100,000 - 120,000 cells/cm² in NS-A medium containing N2 supplement at about 5 µL/ml, B27 supplement at about 10 µL/ml and FGF-2 at about 5 ng/ml for about 3 days in the absence of laminin;
(b) dissociating and re-plating said cells at a density of 14,000 - 18,000 cells/cm² in a 3:1 mix of neurobasal medium (DMEM-F12), containing N2 supplement at about 5 µl/ml, B27 supplement at about 10 µl/ml, FGF-2 at about 5 ng/ml, BDNF at about 20ng/ml and valproic acid at about 1mM and culturing said cells for about 3 days in the presence of laminin; and
(c) transferring said cells to a 3:1 mix of neurobasal medium (DMEM-F 12), containing N2 supplement at about 5 µl/ml, B27 supplement at about 10 µl/ml, FGF-2 at about 3 ng/ml, BDNF at about 30ng/ml and valproic acid at about 1 mM.

Also provided by the invention are cell culture media and kits and uses thereof.

Thus a further aspect of the invention lies in a cell culture medium comprising (1) a neurotrophin, (2) an FGF receptor agonist and (3) a glial differentiation inhibitor.

This medium has been used to obtain cultures with high proportions of neurons showing functionality of *in vivo* neurons and having high viability.

The neurotrophin is present to promote growth of the neural stem cells and their progeny, and preferably is selected from BDNF, NGF, NT3 and NT4. In the examples set out in more detail below, good results have been obtained using BDNF. The FGF receptor agonist is suitably selected from FGF1, FGF2, FGF3, FGF4 and FGF8.

In a specific embodiment, the FGF receptor agonist is FGF-2.

The glial differentiation inhibitor is suitably selected from Trichostatin A, sodium butyrate, suberoylanilide hydroxamic acid, suberoyl-3-aminopyridineamide hyroxamic acid and MS-275. The concentration of these agents is generally so as to prevent differentiation of neural stem cells into glial cells. Trichostatin A can be used at a concentration of from 20 to 150 nM, preferably from 50-75 nM. Sodium Butyrate can be used at a concentration of from 200 - 1500 nM, preferably from 500-750 nM, suberoylanilide hydroxamic acid (SAHA) at from 0.2 to 5 µM, preferably 0.5-1 µM, suroyl-3-aminopyridineamide hydroxamic acid (pyroxamide) from 0.2 to 5 mM, preferably 0.5-1 mM, and MS-275 from 0.2 to 5 mM, preferably from 0.5-1 mM. In an example set out below valproic acid, preferably at a concentration of from 0.2 - 5 mM, more preferably from 0.5 - 2 mM has been used.

The neurotrophin is present in an effective amount. BDNF is suitably at a concentration in the range range 5 - 100 ng/ml, preferably 10-60 ng/ml, more preferably about 20 ng/ml and about 30 ng/ml. NGF can be used at 20-250 ng/ml, preferably 50-100 ng/ml, NT3 at from 20 to 100 ng/ml, preferably about 50 ng/ml and NT4 from 20 to 100 ng/ml, preferably about 50 ng/ml. The medium additionally preferably comprises or is based upon basal medium.

In particular methods of the invention as described elsewhere herein in more detail, culture steps take place using medium in which the ratios of neurotrophin:FGF receptor agonist vary, the ratio changing so that the relative amount of neurotropin increases. Thus in particular aspects of the invention there is provided a set of cell culture media for example as kit components, wherein there is a first medium containing a neurotrophin and an FGF receptor agonist at a first ratio and a second medium containing a neurotrophin and an FGF receptor agonist at a second ratio, wherein the second ratio is at least twice the first ratio. Preferably, the ratio in the first medium is 1-6:1 and the ratio in the second medium is 7-20:1.

Still further provided by the invention are kits for culturing neural stem cells comprising:
(a) a neurotrophin,
(b) an FGF receptor agonist, and
(c) a glial differentiation inhibitor.

The kits may also comprise neural stem cells and/or the multiple media (first and second media) as described immediately above.

The kits can also contain a further medium, being typically a second medium and which can be a third medium if two other media with different ratios of neurotrophin:FGF receptor agonist are present, the further medium being free of neurotrophin, free of glial differentiation inhibitor and containing an agonist of a FGF receptor.

The invention yet further provides a method of preconditioning cells prior to transplantation, comprising:
(a) obtaining a culture of neural stem cells, and
(b) culturing those cells so as to obtain preconditioned cells, wherein the neural cells have the following marker profile:
   - nestin: positive;
   - b III tubulin: negative;
   and the preconditioned cells have the following marker profile:
   - nestin: negative; and
   - b III tubulin: positive.

Expression of a number of markers can be used to indicate the cells are preconditioned in accordance with the invention, and the neural stem cells are preferably also
- vimentin: positive;
and the preconditioned cells have the following marker profile:
- vimentin: negative.
The neural stem cells are preferably also
- RC2: positive;
- b III tubulin: negative;
and the preconditioned cells have the following marker profile:
- RC2: negative.

The preconditioned cells still more preferably have the following marker profile:
- neural filament: negative; and
- MAP2: negative,
and further preferably are
- neun: negative.

In the most preferred embodiment the preconditioned cells have the following marker profile:
- Nestin: negative;
- Tuj 1: positive;
- Doublecortin: positive;
- N-CAM: positive;
- Ki67: negative; and
- Phosphohistone 3: negative.

A more detailed summary of preferred marker profiles is illustrated below:

| **Marker** | **Neural stem cells** | **Preconditioned cells** |
|---|---|---|
| Nestin | positive | negative |
| GFAP | negative | negative |
| Tuj1 | negative | positive |
| Vimentin | positive | negative |
| RC2 | positive | negative |
| 3CB2 | positive | negative |
| Phospho histone 3 | positive | negative |
| BLBP | positive | positive |
| Pax6 | positive | positive |
| Olig2 | positive | positive |
| CyclinD1 | positive | negative |
| N-CAM | negative | positive |
| Doublecortin | negative | positive |
| Ki 67 | positive | negative |
| PCNA | positive | negative |
| ShcA | positive | negative |
| Neurofilaments | positive | positive |
| Map2 | negative | negative |
| NeuN | negative | negative |

To precondition cells it is preferred to culture cells for from 2 to 4 days under differentiating conditions, especially about 3 days under differentiating conditions. In embodiments described further below, preconditioning comprises culturing cells in an agonist of an FGF receptor, typically in medium comprising NS-A medium, N2 supplement, B27 supplement and FGF-2 and further typically comprises plating cells at a density of from 30,000 - 1,200,000 cells/cm ²

An effect of the preconditioning of cells is to render them particularly plastic and suitable for transplantation, e.g. into the adult brain with the capacity to integrate and demonstrate regio-specific differentiation.

A preconditioned cell population of the invention comprises cells derived from neural stem cells, wherein at least 75% of the cells in the preconditioned cell population are negative for the cell markers RC2, vimentin and nestin and positive for the cell marker b III tubulin. Preferably at least 75% of the cells in the preconditioned cells population are negative for the markers RC2, vimentin, and nestin, and positive for the markers b III tubulin. In preferred embodiments, the preconditioned cells have the marker profile set out above.

These cells are suitable for and have been used for transplantation. Hence a method of transplantation of the invention comprises preconditioning cells according to the methods described, and transplanting preconditioned cells. Use of cells preconditioned by the method of the invention in manufacture of a composition for transplantation is another aspect of the invention.

An advantage of the invention is increased yield of neurons from a given starting neural stem cell population and increased survival of neurons - in general cells of the present invention showed 90% expression of type III beta-tubulin at day 10 of culture with 70% survival after two weeks. In a specific example, 100 NS cells differentiated to 90 live cells of which 80% were neuronal. This is a significant improvement over the prior art which obtained only 35-50% neuronal cells.

Neurons produced by the current invention showed high levels of expression of neuronal markers with increased expression of ion channels. They were consequently more like physiological neurons and primary culture cells.

The current invention allows the development of fully mature neurons from ES cells and regions of adult brain.

Cells cultured according the methods of the invention showed stable neurogenesis over time, increased viability and faster maturation.

The invention is now illustrated with reference to the following specific examples.

### EXAMPLES

### Example 1 Improved neuronal differentiation of ES-derived NS cells

We developed a new *in vitro* neuronal differentiation protocol which was found to have increased efficiency comparate with the originally described neuronal differentiation protocols (Conti and Pollard et al., PLoS Biology 2005 (W02005/121318)).

### STEP 1:

NS cells were cultured to 80-90% confluency before being dissociated for 1 minute using Accutase. PBS (5 volumes with respect to Accutase) was added and the cells were centrifuged at 1000g for 3 minutes. The pellet was resuspended, the cells were counted and then plated on an uncoated plastic cell culture flask-dish at 100000-120000 cells/cm ²

Cells were cultured for three days at 37°C in medium comprising: NS-A medium, N2 supplement (5 µL/mL), B27 supplement (10 µL/mL) and FGF-2 (5 ng/mL).

### STEP 2:

The cells were gently dissociated for less than 1 minute using 1 mL of Accutase. PBS (5 volumes with respect to Accutase) was added and the cell suspension was spun at 1000g for 3 minutes. The pellet was resuspended, the cells were then counted and plated on laminin coated (2 µg/mL for 3-5 hours at 37°C) plastic coverslips at 14000-18000 cells/cm².

Cells were then cultured for three days at 37°C in medium (Medium A) comprising; 3:1 mix of Neurobasal medium: DMEM-F 12, N2 supplement (5 µL/mL), B27 supplement (10 µL/mL), FGF-2 (5 ng/mL), BDNF (20ng/mL) and Valproic Acid (Valproate; 1 mM).

### STEP 3 :

The cells were transferred to Medium B comprising; 3:1 mix of Neurobasal medium: DMEM-F 12, N2 supplement (5 µL/mL), B27 supplement (10 µL/mL), FGF-2 (3 ng/mL), BDNF (30ng/mL) and Valproic Acid (Valproate; 1 mM), and cultured for three days at 37°C.

The medium was changed every 3 days with fresh Medium B.

In these conditions 80% of cells expressed neuronal markers such as type III β-tubulin, MAP2 and NeuN *in vitro* with a survival rate of about 90% after 10 days (80% after 2 weeks and 70% after 3 weeks). The improvement was really noticeable with respect to the previous protocol that lead to 35-50% of type III β-tubulin positive cells at 10 days but with a survival rate of approximately 10-20% after two weeks *in vitro.* The newly differentiated NS cells were also positive for further neuronal markers such as Neurofilament M, synapsin and synaptophysin, indicating their late maturation stage.

***Improved neurochemical and functional parameters:*** As a consequence of the improved differentiation, the new NS-derived neurons exhibited an enhanced maturation. Indeed, a massive expression of sodium channels was already evident at 10 days of exposure to the new differentiating conditions, resulting in the generation of sodium currents and leading to action potentials after 15 days *in vitro* (with the prior art protocol action potentials could be detected only after 30 days in vitro). Additional measurements indicated that NS cells exposed to this new differentiation protocol expressed several mature neuronal markers at 14 days.

80% of the neurons in culture were GABAergic as indicated by the GABA immunoreactivity. A fraction (70%) of this GABAergic population was also immunoreactive for GAD65/67.

The neurons also expressed calbindin, calretinin, parvalbumin and voltage-gated channels for sodium and potassium that indeed allowed these neurons to evoke action potentials already at this stage (it was 30 days for the prior art protocol).

The new cultures also expressed GABA receptors alpha and beta and stimulation with specific antagonists was able to induce their functional activation (measured by the appearance of specific currents as assessed by electrophysiology).

### Example 2 Generation of a preconditioned NS cell population that efficiently integrated into the adult brain

NS cells were cultured to 80-90% confluency before being dissociated for 1 minute using Accutase. PBS (5 volumes with respect to Accutase) was added and the cell suspension was centrifuged at 1000g for 3 minutes. The pellet was resuspended, the cells were counted and then plated on an uncoated plastic cell culture flask-dish at 60000 cells/cm ²

Cells were preconditioned by culture for three days at 37°C in medium comprising: NS-A medium, N2 supplement (3.5 µL/mL), B27 supplement (13.5 µL/mL) and FGF-2 (10 ng/mL).

Previous studies indicated that NS cells are able to survive when implanted in the adult mouse brain (Conti and Pollard et al., PLoS Biology 2005 (WO2005/121318)). The cells differentiate both to neurons and astrocytes with similar efficiency (about 40% of neurons and 40% of astrocytes) but without showing a well defined neuronal morphology or final antigenic maturation.

Cells were preconditioned as described and found to have different properties with respect to the known NS cells and were no longer a radial glia population. The preconditioned cells exhibited an extraordinary integration into the host tissue leading to an exclusively neuronal population with a region-specific morphology and antigenic properties. Few or no astrocytic components were observed with more than 90% of the cells having acquired an unequivocally neuronal identity. The results seem to exclude the possibility of fusion events between the grafted NS cells and the endogenous neurons indicating that these results are due to effective neuronal differentiation of the donor neural stem cells. Additionally, these results indicate, without wishing to be bound by any theory, the *ex vivo* preconditioning was able to convert the NS cells into a status which is extremely plastic and able to correctly respond in a region specific manner to environmental cues.

### Example 3

A separate protocol has been custom adjusted for adult SVZ-derived NS cells (in this case, the neuronal differentiation efficiency is about 60% of neurons and 35% of astrocytes; the neurons have the same characteristics of those obtained from the ES-derived NS cells).

The protocol was as in example 1 with the modification that in step 1 the cells were plated directly in DMEM 12 medium rather than NS-A medium.

The invention thus provides neuronal differentiation and transplantation methods and media and kits therefor and therapies based thereon.

## Claims

1. A method of differentiating neural stem cells so as to form neurons, comprising culturing said cells in medium containing a combination of a neurotrophin, an FGF receptor agonist and a glial differentiation inhibitor.

2. The method of claim 1 wherein the neurotrophin is selected from BDNF, NGF, NT3 and NT4.

3. The method of claim 2 wherein the neurotrophin is BDNF.

4. The method of any preceding claim wherein the FGF receptor agonist is selected from FGF 1, FGF2, FGF3, FGF4 and FGF8.

5. The method of claim 4 wherein the FGF receptor agonist is FGF-2.

6. The method of any preceding claim wherein the glial differentiation inhibitor is selected from Trichostatin A, sodium butyrate, suberoylanilide hydroxamic acid, suberoyl-3-aminopyridineamide hyroxamic acid and MS-275.

7. The method of claim 6 wherein the glial differentiation inhibitor is valproic acid.

8. The method of any preceding claim, comprising plating neural stem cells at a density of 30,000 cells/cm² or less.

9. The method of any preceding claim, comprising plating neural stem cells at a density of from 5,000 cells/cm² to 25,000 cells/cm².

10. The method of any preceding claim, comprising plating neural stem cells at a density of from 10,000 cells/cm² to 22,000 cells/cm².

11. A method of differentiating neural stem cells so as to form neurons, comprising a first culture step and a second culture step, wherein the second culture step is a method according to any of claims 1 to 10 and the first culture step comprises culturing the cells in the absence of a neurotrophin, in the absence of a glial differentiation inhibitor and in the presence of an agonist of an FGF receptor.

12. The method of claim 11, comprising plating neural stem cells in the first culture step at a density of at least 50,000 cells/cm ²

13. The method of claim 12, comprising plating neural stem cells in the first culture step at a density of from 50,000 cells/cm² to 200,000 cells/cm².

14. The method of claim 13, comprising plating neural stem cells in the first culture step at a density of from 80,000 cells/cm² to 150,000 cells/cm ²

15. The method of any of claims 11 to 14, wherein the second culture step comprises a first period of culture and a second period of culture, and wherein during the first period the cells are cultured in a ratio of neurotrophin:FGF receptor agonist of from 1:1 to 6:1 and during the second period the cells are cultured in a ratio of neurotrophin:FGF receptor agonist of from 7:1 to 20:1

16. The method of claim 15, wherein during the first period the cells are cultured for from 1 to 5 days.

17. The method of claim 15, wherein during the first period the cells are cultured for from 2 to 4 days.

18. The method of claim 15, wherein during the first period the cells are cultured for about 3 days.

19. The method of any of claims 11 to 18, wherein during the first culture step, the cells are cultured for from 1 to 5 days.

20. The method of any of claims 11 to 18, wherein during the first culture step, the cells are cultured for from 2 to 4 days.

21. A method of differentiating neural stem cells, the method comprising:
(a) culturing neural stem cells in medium containing a neurotrophin and an FGF receptor agonist at a first ratio;
(b) culturing the cells in a medium containing a neurotrophin and an FGF receptor agonist at a second ratio,
wherein the second ratio is at least twice the first ratio.

22. The method of claim 21, comprising:
(a) culturing neural stem cells in medium containing a neurotrophin and an FGF receptor agonist at a ratio of 1-6:1;
(b) culturing neural stem cells in medium containing a neurotrophin and an FGF receptor agonist at a ratio of 7-20:1.

23. The method of claim 21 or 22, wherein both the first and second media contain a glial differentiation inhibitor.

24. The method of claim 23, wherein the glial differentiation inhibitor is valproic acid.

25. The method of any of claims 21 to 24, wherein the cells are cultured for from 1 to 5 days.

26. The method of any of claims 21 to 25, wherein during the cells are cultured for from 2 to 4 days.

27. The method of any of claims 21 to 26, wherein during the cells are cultured for about 3 days.

28. A method of differentiating neural stem cells, the method comprising:
(a) culturing neural stem cells at a density of at least 50,000 cells/cm²;
(b) re-plating said cells at a density of 5000 - 30,000 cells/cm².

29. The method of claim 28, wherein during step (a) the cells are cultured for a period of from 1 to 5 days.

30. The method of claim 28 or 29, wherein during step (b) the cells are cultured for a period of from 1 to 5 days.

31. The method of any of claims 28 to 30, wherein during step (a) the cells are cultured at a density of at least 50,000 cells/cm ²

32. The method of any of claims 28 to 31, wherein during step (a) the cells are cultured at a density of from 50,000 cells/cm² to 200,000 cells/cm².

33. The method of any of claims 28 to 32, wherein during step (a) the cells are cultured at a density of from 80,000 cells/cm² to 150,000 cells/cm².

34. The method of any of claims 28 to 33, wherein during step (b) the cells are cultured at a density of 30,000 cells/cm² or less.

35. The method of any of claims 28 to 34, wherein during step (b) the cells are cultured at a density of from 5,000 cells/cm² to 25,000 cells/cm² .

36. The method of any of claims 28 to 35, wherein during step (b) the cells are cultured at a density of from 10,000 cells/cm² to 22,000 cells/cm ²

37. The method of any of claims 28 to 36, wherein during step (b) the cells are cultured in a medium containing a combination of a neurotrophin, an FGF receptor agonist and a glial differentiation inhibitor.

38. A method of culturing neural stem cells, the method comprising:
(a) culturing neural stem cells at a density of 100,000 - 120,000 cells/cm² in NS-A medium containing N2 supplement, B27 supplement and FGF-2 for from 2 to 4 days;
(b) dissociating the cells and re-plating the cells at a density of 14,000 - 18,000 cells/cm² in neurobasal medium (DMEM-F12), containing N2 supplement, B27 supplement, FGF-2, BDNF and valproic acid and culturing said cells for from 2 to 4 days; and
(c) transferring said cells to neurobasal medium (DMEM-F 12), containing N2 supplement, B27 supplement, FGF-2, BDNF and valproic acid.

39. The method of claim 38, comprising:
(a) culturing neural stem cells at a density of 100,000 - 120,000 cells/cm² in NS-A medium containing N2 supplement at about 5 µL/ml, B27 supplement at about 10 µL/ml and FGF-2 at about 5 ng/ml for about 3 days in the absence of laminin;
(b) dissociating and re-plating said cells at a density of 14,000 - 18,000 celIS/CM² in a 3:1 mix of neurobasal medium (DMEM-F 12), containing N2 supplement at about 5 µl/ml, B27 supplement at about 10 µl/ml, FGF-2 at about 5 ng/ml, BDNF at about 20ng/ml and valproic acid at about 1 mM and culturing said cells for about 3 days in the presence of laminin;
(c) transferring said cells to a 3:1 mix of neurobasal medium (DMEM-F12), containing N2 supplement at about 5 µl/ml, B27 supplement at about 10 µl/ml, FGF-2 at about 3 ng/ml, BDNF at about 30ng/ml and valproic acid at about 1 mM.

40. A cell culture medium comprising (1) a neurotrophin, (2) an FGF receptor agonist and (3) a glial differentiation inhibitor.

41. The medium of claim 40 wherein the neurotrophin is selected from BDNF, NGF, NT3 and NT4.

42. The medium of claim 41 wherein the neurotrophin is BDNF.

43. The medium of any of claims 40 to 42 wherein the FGF receptor agonist is selected from FGF1, FGF2, FGF3, FGF4 and FGF8.

44. The medium of claim 43 wherein the FGF receptor agonist is FGF-2.

45. The medium of any of claims 40 to 44 wherein the glial differentiation inhibitor is selected from Trichostatin A, sodium butyrate, suberoylanilide hydroxamic acid, suberoyl-3-aminopyridineamide hyroxamic acid and MS-275.

46. The medium of claim 45 wherein the glial differentiation inhibitor is valproic acid.

47. The medium of any of claims 40 to 46, further comprising basal medium.

48. A kit for culturing neural stem cells comprising:
(a) a neurotrophin,
(b) an FGF receptor agonist, and
(c) a glial differentiation inhibitor.

49. The kit of claim 48, comprising:
(a) the medium of any of claims 40 to 47; and
(b) neural stem cells.

50. The kit of claim 49, further comprising a second medium, free of neurotrophin, free of glial differentiation inhibitor and containing an agonist of a FGF receptor.

51. A method of preconditioning cells prior to transplantation, comprising:
(a) obtaining a culture of neural stem cells, and
(b) culturing those cells so as to obtain preconditioned cells, wherein the neural cells have the following marker profile:
nestin positive;
b III tubulin negative;
and the preconditioned cells have the following marker profile:
nestin negative; and
b III tubulin positive.

52. The method of claim 51, wherein the neural cells have the following marker profile:
vimentin positive;
nestin positive;
b III tubulin negative;
and the preconditioned cells have the following marker profile:
vimentin negative;
nestin negative; and
b III tubulin positive.

53. The method of claim 51, wherein the neural cells have the following marker profile:
RC2 positive;
vimentin positive;
nestin positive;
b III tubulin negative;
and the preconditioned cells have the following marker profile:
RC2 negative;
vimentin negative;
nestin negative; and
b III tubulin positive.

54. The method of claim 51, wherein the neural cells have the following marker profile:
RC2 positive;
vimentin positive;
nestin positive;
b III tubulin negative;
and the preconditioned cells have the following marker profile:
RC2 negative;
vimentin negative;
nestin negative
b III tubulin positive;
neural filament negative; and
MAP2 negative.

55. The method of claim 51, wherein the neural cells have the following marker profile:
RC2 positive;
vimentin positive;
nestin positive;
b III tubulin negative;
and the preconditioned cells have the following marker profile:
RC2 negative;
vimentin negative;
nestin negative
b III tubulin positive;
neural filament negative;
MAP2 negative; and
neun negative.

56. The method of any of claim 51 to 55, comprising culturing cells for from 2 to 4 days under differentiating conditions.

57. The method of any of claim 51 to 56, comprising culturing cells for about 3 days under differentiating conditions.

58. The method of claim 56 to 57, comprising culturing cells in an agonist of an FGF receptor.

59. The method of any of claims 56 to 58, comprising culturing cells in medium comprising NS-A medium, N2 supplement, B27 supplement and FGF-2.

60. The method of any of claims 51 to 59, comprising plating cells at a density of from 30,000 - 1,200,000 cells/cm².

61. The method of claim 51, wherein the preconditioned cells have the following marker profile:-
nestin negative
Tuj 1 positive
doublecortin positive
N-CAM positive
ki67 negative
phosphohistone 3 negative

62. The method of claim 51, wherein the preconditioned and neural cells have the following marker profile:
| **Marker** | **Neural stem cells** | **Preconditioned cells** |
|---|---|---|
| Nestin | positive | negative |
| GFAP | negative | negative |
| Tuj1 | negative | positive |
| Vimentin | positive | negative |
| RC2 | positive | negative |
| 3CB2 | positive | negative |
| Phospho histone 3 | positive | negative |
| BLBP | positive | positive |
| Pax6 | positive | positive |
| Olig2 | positive | positive |
| CyclinD 1 | positive | negative |
| N-CAM | negative | positive |
| Doublecortin | negative | positive |
| Ki 67 | positive | negative |
| PCNA | positive | negative |
| ShcA | positive | negative |
| Neurofilaments | positive | positive |
| Map2 | negative | negative |
| NeuN | negative | negative |

63. A preconditioned cell population, comprising cells derived from neural stem cells, wherein at least 75% of the cells in the preconditioned cell population are negative for the cell markers RC2, vimentin and nestin and positive for the cell marker b III tubulin.

64. The cell population of claim 63, wherein at least 75% of the cells in the preconditioned cells population are negative for the markers RC2, vimentin, and nestin, and positive for the markers b III tubulin.

65. A preconditioned cells population, prepared according to the method of any of claims 51 to 62.

66. A preconditioned cells population having a marker profile as set out in claim 61 or 62.

67. A method of transplantation, comprising preconditioning cells according to the method of any of claims 51 to 62, and transplanting preconditioned cells.

68. The method of claim 67, comprising transplanting the cells into the brain of a human.

69. Use of cells preconditioned by the method of any of claims 51 to 62 in manufacture of a composition for transplantation.
